# EUROPÄISCHE PATENTSCHRIFT

(11) **EP 0 738 258 B1**
(45) Veröffentlichungstag und Bekanntmachung des Hinweises auf die Patenterteilung: **28.07.1999**
(21) Anmeldenummer: 95905570.8
(22) Anmeldetag: 21.12.1994
(51) Int. Cl.: C07C 227/08, C07C 229/18, C07C 229/14, C07D 209/36

(54) **VERFAHREN ZUR HERSTELLUNG VON N-SUBSTITUIERTEN GLYCINSÄUREN ODER GLYCINESTERN UND VERWENDUNG DES VERFAHRENS ZUR INDIGOSYNTHESE**
PROCESS FOR PREPARING N-SUBSTITUTED GLYCINIC ACIDS OR GLYCINE ESTERS AND USE OF SAID PROCESS IN INDIGO SYNTHESIS
PROCEDE DE PREPARATION D'ACIDES GLYCINIQUES OU D'ESTERS DE GLYCINE N-SUBSTITUES ET UTILISATION DE CE PROCEDE POUR LA SYNTHESE D'INDIGO

(30) Priorität: 27.12.1993 AT 262593
(43) Veröffentlichungstag der Anmeldung: 23.10.1996
(73) Patentinhaber: DSM Fine Chemicals Austria GmbH, 4021 Linz (AT)
(72) Erfinder: KOS, Carlo, A-4060 Leonding (AT)
(74) Vertreter: Klostermann, Ingrid
(86) Internationale Anmeldenummer: EP9404259
(87) Internationale Veröffentlichungsnummer: WO9518093

(56) Entgegenhaltungen:
- EP-A- 0 036 161
- EP-A- 0 099 981
- US-A- 4 073 804

## Beschreibung

Indigo wird weltweit seit langem aus N-Phenylglycin, das über das Indoxyl zum Indigo oxidiert wird, hergestellt. Gemäß Ullmann, Vol. A 14, 149 bis 156 wird das dazu nötige N-Phenylglycin im wesentlichen entweder durch Umsetzung von Monochloressigsäure mit Anilin oder Anthranilsäure oder durch Hydrolyse von N-Cyanomethylanilin hergestellt. Zur Herstellung von N-Cyanomethylanilin wird dabei Blausäure oder Natriumcyanid mit Dianilinomethan reagieren gelassen. Das Arbeiten mit Monochloressigsäure oder mit Cyaniden ist aber aus Sicherheits- und Umweltschutzgründen nicht erwünscht.

Es wurde nun unerwarteterweise gefunden, daß Indoxyl nicht nur aus N-Phenylglycin, sondern auch aus N-Phenylglycinestern hergestellt werden kann und daß N-substituierte Glycinester und auch N-substituierte Glycinsäuren auf umweltfreundlichem Weg durch Umsetzung eines Amins mit einem Glyoxylsäureesterhalbacetal herstellbar sind. Glyoxylsäureesterhalbacetale können großtechnisch auf umweltfreundlichem Weg durch Ozonolyse von Maleinsäurederivaten und Hydrieren der peroxidischen Reaktionslösung etwa gemäß EP B-0 099 981 erzeugt werden.

Ein Verfahren zur Herstellung von Glycin ist in US 4,073,804 beschrieben, bei welchem Glyoxylsäure in einem Gemisch aus Wasser und organischem Lösungsmittel mit Ammoniak in Gegenwart eines Rhodiumkatalysators umgesetzt wird.

Gegenstand der Erfindung ist daher ein Verfahren zur Herstellung von N-substituierten Glycinsäuren oder Glycinestern der Formel

R₁R₂N-CH₂-COOR I,

in der R eine geradkettige oder verzweigte Alkylgruppe mit 1 bis 10 C-Atomen und R₁ und R₂ unabhängig voneinander Wasserstoff, eine unsubstituierte oder durch Halogen, durch Alkylgruppen mit 1 bis 6 C-Atomen oder durch Alkoxygruppen mit 1 bis 4 C-Atomen substituierte Phenyl- oder Naphthylgruppe oder eine geradkettige, verzweigte oder cyclische Alkylgruppen mit 1 bis 22 C-Atomen, die unsubstituiert oder durch Alkoxygruppen mit 1 bis 4 C-Atomen oder Phenylgruppen substituiert sein können bedeuten, wobei R₁ und R₂ nicht gleichzeitig Wasserstoff bedeuten, das dadurch gekennzeichnet ist, daß ein Glyoxylsäureesterhalbacetal der Formel

R₃O(OH)CH - COOR II,

in der R die obgenannte Bedeutung hat und R₃ eine Alkylgruppe mit 1 bis 4 C-Atomen bedeutet, mit einem Amin der Formel

R₁R₂NH III,

in der R₁ und R₂ die obgenannte Bedeutung haben in einem Verdünnungsmittel bei Temperaturen von 0°C bis zur Rückflußtemperatur des verwendeten Verdünnungsmittels umgesetzt und das Umsetzungsprodukt in Gegenwart eines Hydrierkatalysators und eines Verdünnungsmittels mit Wasserstoff unter Druck behandelt wird, wobei der N-substituierte Glycinester der Formel I entsteht, der falls erwünscht, aus dem Reaktionsgemisch isoliert und gegebenenfalls in ein Salz oder in die freie Säure überführt wird.

In den Formeln I bis III bedeuten R eine geradkettige oder verzweigte Alkylgruppe mit 1 bis 10 C-Atomen, bevorzugt mit 1 bis 4 C-Atomen, ganz bevorzugt mit 1 oder 2 C-Atomen , R₃ eine Alkylgruppe mit 1 bis 4 C-Atomen, bevorzugt mit 1 bis 2 C-Atomen , wobei besonders bevorzugt R und R₃ die gleiche Bedeutung haben. R₁ und R₂ bedeuten unabhängig voneinander Wasserstoff, eine Alkylgruppe oder eine Arylgruppe, wobei R₁ und R₂ nicht gleichzeitig Wasserstoff bedeuten. Unter Alkylgruppe ist dabei eine geradkettige, verzweigte oder cyclische Alkylgruppe mit 1 bis 22 C-Atomen zu verstehen, die unsubstituiert oder durch Alkoxygruppen mit 1 bis 4 C-Atomen oder durch Phenylgruppen substituiert sein kann, beispielsweise Methyl-, Ethyl-, n-Propyl-, Butyl-, Octyl-, Dodecyl-, Hexadecylgruppe oder deren Isomeren wie iso-Propyl-, iso-Butyl-, 2-Ethylhexyl-, iso-Dodecylgruppen oder Benzyl-, Ethylphenylgruppen. Alkoxygruppen sind beispielsweise Methoxy-, Ethoxy-, Butoxy-, iso-Butoxygruppen. Eine Arylgruppe ist eine unsubstituierte oder durch Halogen, durch Alkylgruppen bevorzugt mit 1 bis 6 C-Atomen, besonders bevorzugt mit 1 bis 4 C-Atomen, durch Alkoxygruppen bevorzugt mit 1 bis 4 C-Atomen substituierte Phenyl- oder Naphthylgruppe, wobei eine Phenylgruppe bevorzugt ist. Halogen bedeutet Fluor, Chlor oder Brom. Bevorzugt bedeutet R₁ Wasserstoff und R₂ eine Arylgruppe, bevorzugt eine unsubstituierte oder durch Halogen, durch Alkylgruppen mit 1 bis 4 C-Atomen oder durch Alkoxygruppen mit 1 bis 4 C-Atomen, besonders bevorzugt eine unsubstituierte oder durch Alkylgruppen substituierte Phenyl- oder Naphthylgruppe.

Zu den bevorzugten N-substituierten Glycinsäuren und Glycinestern zählen demnach Verbindungen der Formel 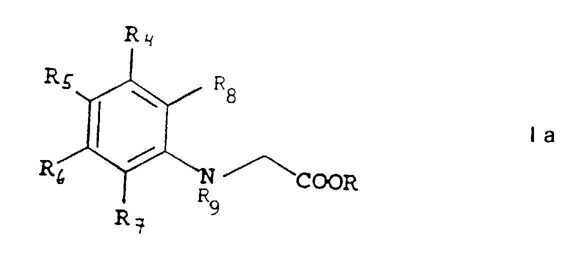 in der R die in der Formel I angegebene Bedeutung hat, R₄, R₅, R₆, R₇ und R₈ unabhängig voneinander Wasserstoff, Halogen, Alkylgruppen mit 1 bis 6 C-Atomen oder Alkoxygruppen mit 1 bis 4 C-Atomen oder R₅ und R₆ oder R₆ und R₇ oder R₄ und R₈ gemeinsam mit den jeweiligen beiden C-Atomen an die sie substituiert sind, einen unsubstituierten oder durch Halogen, Alkylgruppen mit 1 bis 6 C-Atomen oder Alkoxygruppen mit 1 bis 4 C-Atomen substituierten Benzolring bedeuten und R₉ Wasserstoff, einen Alkylrest mit 1 bis 6 C-Atomen, der gegebenenfalls substituiert sein kann, oder einen Arylrest, der gegebenenfalls durch Halogen, Alkylgruppen oder Alkoxygruppen substituiert sein kann, bedeutet.

Zur Herstellung der Verbindungen der Formel I wird zuerst ein Glyoxylsäureesterhalbacetal der Formel II mit einem Amin der Formel III in einem bei Temperaturen von 0°C, vorzugsweise von Raumtemperatur bis zur Rückflußtemperatur des verwendeten Verdünnungsmittels gegebenenfalls unter Druck erhitzt und reagieren gelassen. Unter Verdünnungsmittel sind bevorzugt Alkohole wie Methanol, Ethanol, iso-Propanol, Butanol zu verstehen. Bevorzugt wird jener Alkohol, in dem der Alkylteil den Alkylteilen im verwendeten Glyoxylsäureesterhalbacetal entspricht, als Verdünnungsmittel eingesetzt.
Pro Mol Glyoxylsäureesterhalbacetal der Formel II werden dabei mindestens 1 Mol, im allgemeinen aber 1 bis 5 Mol, bevorzugt 1 bis 3 Mol, besonders bevorzugt 1 bis 1,5 Mol Amin der Formel III eingesetzt. Bevorzugt erfolgt die Reaktion unter Normaldruck, wobei aber Drücke von 1 bis 20 bar angewendet werden können.
Die Reaktion wird wie üblich, bevorzugt chromatographisch verfolgt. Nach beendeter Reaktion, die durch das Verschwinden des jeweiligen Halbacetals aus dem Reaktionsgemisch festgestellt wird, wird die Reaktionsmischung abgekühlt. Die gebildete Zwischenverbindung, die chemisch nicht identifiziert wurde, die aber vermutlich eine Verbindung der Formel R₁R₂-N-C(OH)(OR₃)-COOR, R₁R₂-N-CH(OH)-COOR, R₁R₂-N-CH(OR₃)-COOR oder im Falle daß beispielsweise R₂ ein Wasserstoffatom bedeutet, eine Verbindung der Formel R₁N=CH-COOR, wobei R, R₁, R₂ und R₃ in den genannten Verbindungen die obgenannte Bedeutung haben, darstellen dürfte, kann durch Abdampfen des Verdünnungsmittels isoliert und gegebenenfalls, etwa mit Hilfe von Extraktion, Destillation, Chromatographie gereinigt werden. Es hat sich aber als vorteilhaft herausgestellt, daß die Reaktionsmischung direkt und ohne Isolierung des Zwischenproduktes der Hydrierung unterworfen werden kann.

Die Hydrierung der Zwischenverbindung erfolgt mit Hilfe von Wasserstoff in einem Verdünnungsmittel in Gegenwart eines Hydrierkatalysators. Als Verdünnungsmittel eignen sich Verdünnungsmittel, die unter den Reaktionsbedingungen inert sind, beispielsweise aliphatische Kohlenwasserstoffe, wie Hexan, Pentan, aromatische Kohlenwasserstoffe, wie Toluol, Xylole, Ether, wie iso-Propylether, Methyl-tert. Butylether, Tetrahydrofuran, Dioxan, Pyridin, Wasser und Alkohole oder Mischungen solcher Verdünnungsmittel, bevorzugt aliphatische Alkohole mit 1 bis 8 C-Atomen, beispielsweise Methanol, Ethanol, iso-Propanol, Butanol, Hexol, Oktanol. Das Verdünnungsmittel wird im Überschuß zur Zwischenverbindung, bevorzugt im 5 bis 30-fachen Überschuß bezogen auf das Gewicht eingesetzt. Die Zwischenverbindung muß im Verdünnungsmittel löslich sein.
Als Hydrierkatalysator dienen Katalysatoren, die geeignet sind, die Abspaltung von Halbacetal-, Hydroxy- oder Alkoxygruppen von einem C-Atom unter Wasserstoffzufuhr zu katalysieren, bzw. die geeignet sind, die Hydrierung von Enaminen zu Aminen zu katalysieren. Solche Katalysatoren weisen als aktive Komponente Metalle wie beispielsweise Nickel, Kobalt, Platin, Palladium oder chemische Verbindungen solcher Metalle auf, beispielsweise Oxide, die miteinander und/oder mit anderen Metallen oder Metallverbindungen, beispielsweise Eisen, Rhodium, Kupfer legiert, durchsetzt, oder beschichtet sein können. Bevorzugt enthält der Katalysator Nickel als aktiven Bestandteil. Der Katalysator kann dabei als solcher, aufgebracht auf ein übliches Trägermaterial oder auf einen monolithischen Träger, gegebenenfalls als Festbettkatalysator eingesetzt werden und wird bevorzugt aufgebracht auf einen Träger eingesetzt.
Im allgemeinen werden pro Mol Zwischenverbindung mindestens 0,5 g Katalysator verwendet. Da die optimale Menge des Katalysators von seiner Effektivität abhängt, kann es aber von Vorteil sein, größere oder kleinere Katalysatormengen einzusetzen. Der optimale Katalysator und die optimale Katalysatormenge können leicht durch einfache Vorversuche mit verschiedenen Mengen von Katalysatoren bekannter Spezifität ermittelt werden.
Der Wasserstoff wird wie üblich in die Reaktionsmischung eingebracht, vorteilhafterweise wird der Wasserstoff auf die Reaktionsmischung, bestehend aus Zwischenverbindung, Verdünnungsmittel und Hydrierkatalysator aufgedrückt. Dabei wird ein Wasserstoffdruck von 1 bis 120 bar, bevorzugt von 20 bis 100 bar , besonders bevorzugt von 40 bis 80 bar eingestellt.
Die Hydrierung wird bei Temperaturen von etwa 10°C bis etwa 150°C, bevorzugt etwa zwischen 20°C und 130°C durchgeführt.

Dabei bildet sich der N-substituierte Glycinester der Formel I in hohen Ausbeuten. Die Reaktion wird mit Hilfe geeigneter Methoden, bevorzugt chromatographisch verfolgt. Nach beendeter Reaktion kann der N-substituierte Glycinester aus dem Reaktionsgemisch durch Abdampfen des Verdünnungsmittels isoliert und gegebenenfalls mit Hilfe üblicher Methoden, wie Extraktion, Chromatographie, Destillation gereinigt werden.
Im allgemeinen ist die Reinheit des erhaltenen N-substituierten Glycinesters jedoch sehr hoch und daher für die meisten Zwecke ohne Reinigungsschritt ausreichend. Die Reaktionsmischung, die beispielsweise den N-Phenylglycinester enthält, kann daher direkt in eine beliebige Weiterreaktion eingesetzt werden.

Der N-substiuierte Glycinester kann gegebenenfalls in bekannter Weise in ein Salz, beispielsweise ein Alkali- oder Erdalkalisalz überführt werden. Bevorzugte Salze sind das Na- und das K-Salz. Der Glycinester kann weiters, falls gewünscht, in bekannter Weise in die freie Säure überführt werden.

In einer besonders bevorzugten Ausführungsform des Verfahrens werden Glyoxylsäureesterhalbacetal, worin die beiden Alkylgruppen gleich sind und jeweils eine geradkettige Alkylgruppe mit 1 bis 4 C-Atomen bedeuten, mit einem Amin der Formel R₁NH₂, in der R₁ eine unsubstituierte oder durch Halogen, durch Alkylgruppen mit 1 bis 4 C-Atomen oder durch Alkoxygruppen mit 1 bis 4-C-Atomen substituierte Phenyl- oder Naphthylgruppe bedeutet im Molverhältnis 1 zu 1 bis 1,5 in einem Alkylalkohol, in dem der Alkylteil den Alkylteilen im eingesetzten Glyoxylsäureesterhalbacetal entspricht, bei Normaldruck auf Rückflußtemperatur erhitzt. Die Reaktion wird chromatographisch verfolgt. Nachdem das Glyoxylsäureesterhalbacetal aus der Reaktionsmischung verschwunden ist, wird ein Hydrierkatalysator, der als aktiven Bestandteil Nickel enthält und der auf einen Träger aufgebracht ist, in die Reaktionsmischung eingebracht und Wasserstoff mit einem Druck von 40 bis 80 bar aufgedrückt. Die Reaktion wird chromatographisch verfolgt. Nach beendeter Reaktion wird der gebildete N-Phenylglycinester gegebenenfalls durch Abdampfen des Verdünnungsmittels isoliert und gegebenenfalls, wie üblich, etwa durch Extraktion, Destillation, Chromatographie weiter gereinigt oder die Reaktionsmischung, die den N-Phenylglycinester enthält, wird direkt in einer beliebigen Weiterreaktion eingesetzt.

Die N-substituierte Glycinester der Formel I können zur Synthese verschiedenster chemischer Verbindungen, etwa für Zwischenprodukte für Herbizide, für Synthone oder pharmazeutische Zwischenprodukte, verwendet werden. N-Arylglycinester, hergestellt nach dem erfindungsgemäßen Verfahren werden bevorzugt zur Herstellung entsprechender Indoxyl- und weiter zur Herstellung entsprechender Indigoderivate eingesetzt. Es hat sich nämlich unerwarteterweise herausgestellt, daß es möglich ist, den Ringschluß eines N-Arylglycinesters zum Indoxyl direkt, das heißt ohne vorherige Verseifung der Estergruppen vorzunehmen.

Gegenstand der Erfindung ist daher auch ein Verfahren zur Herstellung eines Indoxylderivates der Formel 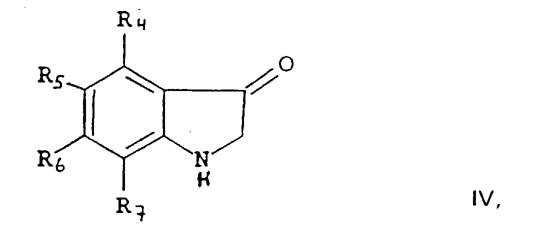 in der R₄, R₅, R₆, und R₇ unabhängig voneinander Wasserstoff, Halogen, Alkylgruppen mit 1 bis 6 C-Atomen oder Alkoxygruppen mit 1 bis 4 C-Atomen oder R₅ und R₆ oder R₆ und R₇ gemeinsam mit den jeweiligen beiden C-Atomen, an denen sie substiuiert sind, einen unsubstituierten oder durch Halogen, Alkylgruppen mit 1 bis 6 C-Atomen oder Alkoxygruppen mit 1 bis 4 C-Atomen substituierten Benzolring bedeuten, das dadurch gekennzeichnet ist, daß ein N-Arylglycinester der Formel 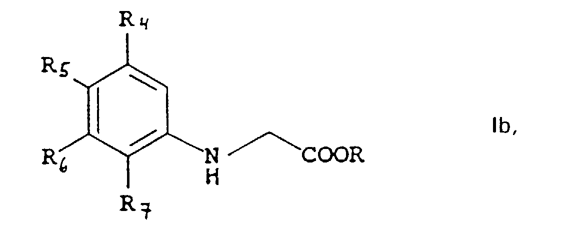 in der R die in der Formel I angegebene Bedeutung hat und R₄, R₅, R₆, und R₇ die obgenannte Bedeutung haben wie oben beschrieben hergestellt und mit oder ohne Isolierung in Gegenwart von geschmolzenem Alkali- oder Erdalkalihydroxid mit oder ohne Zusatz eines Alkaliamids bei Temperaturen von 150 bis 300°C zum Indoxyl der Formel IV ringgeschlossen wird.

Bevorzugt bedeuten in der Formel IV R₄, R₅, R₆ und R₇ Wasserstoff, Halogen, Alkylgruppen mit 1 bis 4 C-Atomen, oder Alkoxygruppen mit 1 bis 4 C-Atomen, besonders bevorzugt Wasserstoff oder Alkylgruppen.
Der Ringschluß des N-Arylglycinesters kann dabei unerwarteterweise wie bei Arylglycinen selbst, etwa gemäß Römpps Chemie Lexikon, Seite 1861 ff, beschriebenen Art und Weise erfolgen, wobei die Alkylestergruppe des N-substituierten Glycinesters als Alkohol abgespalten wird.

Gegenstand der Erfindung ist weiterhin ein Verfahren zur Herstellung eines Indigoderivates der Formel 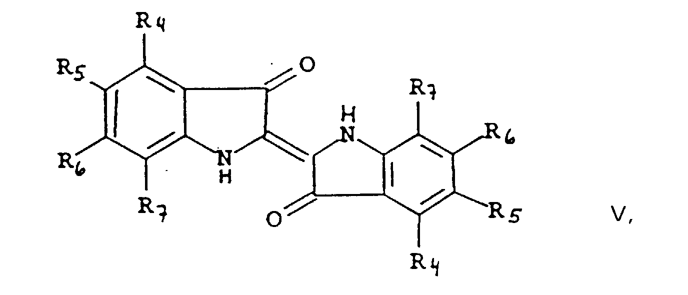 in der R₄, R₅, R₆ und R₇ die in Formel IV angegebene Bedeutung haben, das dadurch gekennzeichnet ist, daß ein Indoxylderivat der Formel IV, in der R₄, R₅, R₆ und R₇ die obgenannte Bedeutung haben erfindungsgemäß hergestellt und auf übliche Art und Weise, bevorzugt etwa gemäß Chemische Berichte, Jg. 99, 1966, Seiten 2146 - 2154, zum Indigoderivat der Formel V oxidiert wird.

Auf die beschriebene Art und Weise können N-substituierte Glycinester, Indoxyl- und Indigoverbindungen auf umweltfreundlichem Weg in guten Ausbeuten hergestellt werden. Das erfindungsgemäße Verfahren stellt daher eine Bereicherung der Technik dar.

### Beispiele

Allgemeine Herstellungsvorschrift:
0,375 Mol des Amins der Formel III wurden in etwa der 9 bis 13-fachen Gewichtsmenge Methanol gelöst, mit 0,375 Mol Glyoxylsäuremethylestermethylhemiacetal (GMHA) (45 g) gelöst in etwa der 12-fachen Gewichtsmenge Methanol, versetzt und bei 25 bis 45°C reagieren gelassen. Der Fortgang der Reaktion wurde mittels Dünnschichtchromatographie verfolgt. Nach beendeter Reaktion wurde die Reaktionslösung in einen Hydrierreaktor eingespeist und bei einer Temperatur von etwa 115°C und einem Wasserstoffdruck von etwa 60 bar mittels eines Nickelkatalysators, der auf einen Träger aufgebracht war (Ni 6458 der Fa. Engelhardt), der Hydrogenolyse unterworfen. Die Reaktion wurde dünnschichtchromatographisch verfolgt. Nach beendeter Reaktion wurde der Katalysator abfiltriert und das Verdünnungsmittel abdestilliert. Da die Reinheit der dabei erhaltenen N-substituierten Glycinester sehr hoch war und in einigen Fällen 99% überstieg, konnte auf eine weitere Reinigung verzichtet werden. In Tabelle 1 sind der allgemeinen Herstellungsvorschrift entsprechend hergestellte N-substituierte Glycinester und die erhaltenen Ausbeuten angeführt. Die Charakterisierung der erhaltenen N-substituierten Glycinester und deren Reinheit wurden gaschromatographisch, durch Vergleich mit den chemisch reinen Substanzen durchgeführt.

**Tabelle 1**

| **Beispiel** | **A** | **B** | **P** | **Ausbeute** |
|---|---|---|---|---|
| 1 | 34,8 g Anilin | 45 g GMHA | 62 g N-Phenylglycinsäuremethylester | 100 |
| 2 | 40,9 g 4-Hydroxyanilin | 45 g GMHA | 67 g N-4-Hydroxyphenylglycinsäuremethylester | 99 |
| 3 | 56,6 g Anthranilssäuremethylester | 45 g GMHA | 56 g N-2-Carbomethoxy-phenylglycinsäuremethylester | 99 |
| 4 | 53,6 g 1-Naphthylamin | 45 g GMHA | 62 g N-Naphthylglycinsäuremethylester | 69 |
| 5 | 40,1 g Benzylamin | 45 g GMHA | 67 g N-Benzylglycinsäuremethylester | 100 |
| In der Tabelle 1 bedeuten **A**: Menge und Art des Amins der Formel III **B**: Menge an Glyoxylsäureesterhalbacetal **P**: Menge und Art des als Produkt erhaltenen N-substituierten Glycinesters **Ausb.:** Ausbeute in % der Theorie bezogen auf das eingesetzte Amin der Formel III | | | | |

### Beispiel 6

0,5 g N-Phenylglycinsäuremethylester, hergestellt in der in Beispiel 1 beschriebenen Art und Weise, wurden in eine heiße Schmelze von 2,5 g Kaliumhydroxid, in die 0,3 g Natriumamid eingebracht waren, und die eine Temperatur von etwa 260 bis 270°C aufwies, eingebracht und einige Minuten ragieren gelassen. Die dabei entstandene Indoxyl - Kaliumhydroxid Schmelze wurde in Eiswasser eingetragen. Durch Einbringen von Luft in die wäßrige Suspension, Abfiltrieren und Trocknen des gebildeten Niederschlages wurden 0,3 g Indigo erhalten.

## Patentansprüche

1. Verfahren zur Herstellung eines N-substituierten Glycinesters oder einer N-substituierten Glycinsäure der Formel
R₁R₂N - CH₂ - COOR I,
in der R eine geradkettige oder verzweigte Alkylgruppe mit 1 bis 10 C-Atomen und R₁ und R₂ unabhängig voneinander Wasserstoff, eine unsubstituierte oder durch Halogen, durch Alkylgruppen mit 1 bis 6 C-Atomen oder durch Alkoxygruppen mit 1 bis 4 C-Atomen substituierte Phenyl- oder Naphthylgruppe oder eine geradkettige, verzweigte oder cyclische Alkylgruppen mit 1 bis 22 C-Atomen, die unsubstituiert oder durch Alkoxygruppen mit 1 bis 4 C-Atomen oder Phenylgruppen substituiert sein können bedeuten, wobei R₁ und R₂ nicht gleichzeitig Wasserstoff bedeuten, dadurch gekennzeichnet, daß ein Glyoxylsäureesterhalbacetal der Formel
R₃O(OH)CH - COOR II,
in der R die obgenannte Bedeutung hat und R₃ eine Alkylgruppe mit 1 bis 4 C-Atomen bedeutet, mit einem Amin der Formel
R₁R₂NH III,
in der R₁ und R₂ die obgenannte Bedeutung haben in einem Verdünnungsmittel bei Temperaturen von 0°C bis zur Rückflußtemperatur des verwendeten Verdünnungsmittels umgesetzt und das gebildete Zwischenprodukt in Gegenwart eines Hydrierkatalysators und eines Verdünnungsmittels mit Wasserstoff unter Druck behandelt wird, wobei der N-substituierte Glycinester der Formel I entsteht, der falls erwünscht, aus dem Reaktionsgemisch isoliert und gegebenenfalls in ein Salz, oder in die freie Säure überführt wird.

2. Verfahren nach Anspruch 1, dadurch gekennzeichnet, daß eine Verbindung der Formel II, in der R und R₃ gleich sind und Alkylgruppen mit 1 bis 4 C-Atomen bedeuten, eingesetzt wird.

3. Verfahren nach einem der Ansprüche 1 oder 2, dadurch gekennzeichnet, daß eine Verbindung der Formel III, in der R₁ eine unsubstituierte oder durch Halogen, durch Alkylgruppen mit 1 bis 6 C-Atomen oder durch Alkoxygruppen mit 1 bis 4 C-Atomen substituierte Phenyl- oder Naphthylgruppe und R₂ Wasserstoff bedeutet, eingesetzt wird.

4. Verfahren nach einem der Ansprüche 1 bis 3, dadurch gekennzeichnet, daß bei der Hydrogenolyse ein Druck von 40 bis 80 bar und eine Temperatur von 20 bis 130°C aufrechterhalten wird.

5. Verfahren nach einem der Ansprüche 1 bis 4, dadurch gekennzeichnet, daß eine Verbindung der Formel III, in der R₁ eine unsubstituierte oder durch Alkylgruppen mit 1 bis 4 C-Atomen substituierte Phenyl- oder Naphthylgruppe und R₂ Wasserstoff bedeutet, eingesetzt wird.

6. Verfahren nach einem der Ansprüche 1 bis 5, dadurch gekennzeichnet, daß pro Mol der Verbindung II 1 bis 1,5 Mol der Verbindung der Formel III eingesetzt wird.

7. Verfahren nach einem der Ansprüche 1 bis 6, dadurch gekennzeichnet, daß als Verdünnungsmittel ein aliphatischer Alkohol mit 1 bis 4 C-Atomen, dessen Alkylteil den Alkylteilen im eingesetzten Glyoxylsäureesterhalbacetal entspricht, eingesetzt wird.

8. Verfahren zur Herstellung eines Indoxylderivates der Formel 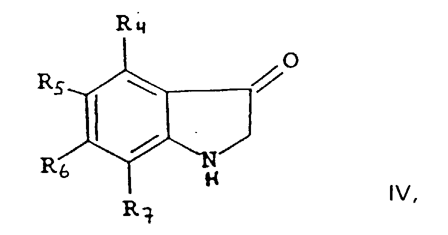 in der R₄, R₅, R₆ und R₇ unabhängig voneinander Wasserstoff, Halogen, Alkylgruppen mit 1 bis 6 C-Atomen oder Alkoxygruppen mit 1 bis 4 C-Atomen oder R₅ und R₆ oder R₆ und R₇ gemeinsam mit den jeweiligen beiden C-Atomen, an denen sie substiuiert sind, einen unsubstituierten oder durch Halogen, Alkylgruppen mit 1 bis 6 C-Atomen oder Alkoxygruppen mit 1 bis 4 C-Atomen substituierten Benzolring bedeuten, das dadurch gekennzeichnet ist, daß ein N-Arylglycinester der Formel 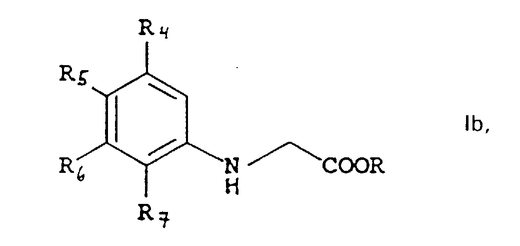 in der R die in der Formel I nach Anspruch 1 angegebene Bedeutung hat und R₄, R₅, R₆ und R₇ die obgenannte Bedeutung haben nach einem der Ansprüche 1 bis 6 hergestellt und in Gegenwart von geschmolzenem Alkali- oder Erdalkalihydroxid mit oder ohne Zusatz eines Alkaliamids bei Temperaturen von 150 bis 300°C zum Indoxyl der Formel IV ringgeschlossen wird.

9. Verfahren nach Anspruch 8, dadurch gekennzeichnet, daß ein N-Arylglycinester der Formel la, in der R eine Alkylgruppe mit 1 bis 4 C-Atomen und R₄, R₅, R₆ und R₇ unabhängig voneinander Wasserstoff, Halogen, Alkylgruppen mit 1 bis 4 C-Atomen oder Alkoxygruppen mit 1 bis 4 C-Atomen bedeuten, eingesetzt wird.

10. Verfahren zur Herstellung eines Indigoderivates der Formel 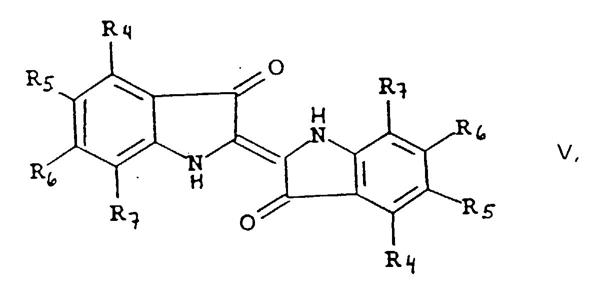 in der R₄, R₅, R₆ und R₇ die in Formel IV nach Anspruch 8 angegebene Bedeutung haben, dadurch gekennzeichnet, daß ein Indoxylderivat der Formel IV nach Anspruch 8 gemäß Anspruch 8 hergestellt und auf übliche Art und Weise zum Indigoderivat der Formel V oxidiert wird.

## Claims

1. Process for the preparation of an N-substituted glycine ester or an N-substituted glycine acid of the formula
R₁R₂N - CH₂ - COOR I
in which R is a straight-chain or branched alkyl group having 1 to 10 C atoms and R₁ and R₂ independently of one another are hydrogen, a phenyl or naphthyl group which is unsubstituted or substituted by halogen, by alkyl groups having 1 to 6 C atoms or by alkoxy groups having 1 to 4 C atoms, or a straight-chain, branched or cyclic alkyl groups having 1 to 22 C atoms which can be unsubstituted or substituted by alkoxy groups having 1 to 4 C atoms or phenyl groups, where R₁ and R₂ are not simultaneously hydrogen, characterized in that a glyoxylic acid ester half-acetal of the formula
R₃O(OH)CH - COOR II
in which R has the abovementioned meaning and R₃ is an alkyl group having 1 to 4 C atoms, is reacted with an amine of the formula
R₁R₂NH III
in which R₁ and R₂ have the abovementioned meaning, in a diluent at temperatures from 0°C up to the reflux temperature of the diluent used, and the resulting intermediate product is treated with hydrogen under pressure in the presence of a hydrogenation catalyst and a diluent, the N-substituted glycine ester of the formula I being formed, which if desired, is isolated from the reaction mixture and if appropriate converted into a salt, or into the free acid.

2. Process according to Claim 1, characterized in that a compound of the formula II in which R and R₃ are identical and are alkyl groups having 1 to 4 C atoms is employed.

3. Process according to one of Claims 1 or 2, characterized in that a compound of the formula III in which R₁ is a phenyl or naphthyl group which is unsubstituted or substituted by halogen, by alkyl groups having 1 to 6 C atoms or by alkoxy groups having 1 to 4 C atoms and R₂ is hydrogen is employed.

4. Process according to one of Claims 1 to 3, characterized in that a pressure of 40 to 80 bar and a temperature of 20 to 130°C is maintained during the hydrogenolysis.

5. Process according to one of Claims 1 to 4, characterized in that a compound of the formula III in which R₁ is a phenyl or naphthyl group which is unsubstituted or substituted by alkyl groups having 1 to 4 C atoms and R₂ is hydrogen is employed.

6. Process according to one of Claims 1 to 5, characterized in that 1 to 1.5 mol of the compound of the formula III are employed per mole of the compound II.

7. Process according to one of Claims 1 to 6, characterized in that an aliphatic alcohol having 1 to 4 C atoms, the alkyl part of which corresponds to the alkyl parts in the glyoxylic acid ester half-acetal employed, is employed as the diluent.

8. Process for the preparation of an indoxyl derivative of the formula 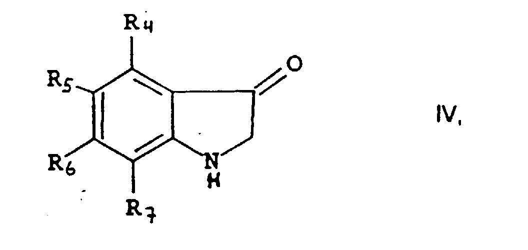 in which R₄, R₅, R₆ and R₇ independently of one another are hydrogen, halogen, alkyl groups having 1 to 6 C atoms or alkoxy groups having 1 to 4 C atoms, or R₅ and R₆ or R₆ and R₇, together with the two particular C atoms on which they are substituted, are a benzene ring which is unsubstituted or substituted by halogen, alkyl groups having 1 to 6 C atoms or alkoxy groups having 1 to 4 C atoms, which is characterized in that an N-arylglycine ester of the formula 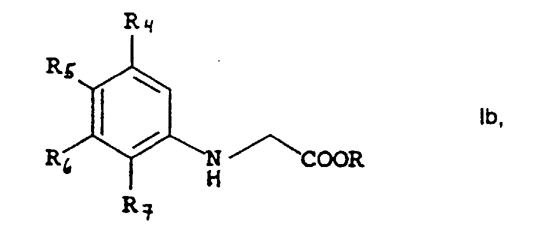 in which R has the meaning given in the formula I according to Claim 1 and R₄, R₅, R₆ and R₇ have the abovementioned meaning, according to one of Claims 1 to 6 is prepared and cyclized, in the presence of molten alkali metal hydroxide or alkaline earth metal hydroxide, with or without addition of an alkali metal amide, at temperatures from 150 to 300°C to give the indoxyl of the formula IV .

9. Process according to Claim 8, characterized in that an N-arylglycine ester of the formula Ia in which R is an alkyl group having 1 to 4 C atoms and R₄, R₅, R₆ and R₇ independently of one another are hydrogen, halogen, alkyl groups having 1 to 4 C atoms or alkoxy groups having 1 to 4 C atoms is employed.

10. Process for the preparation of an indigo derivative of the formula 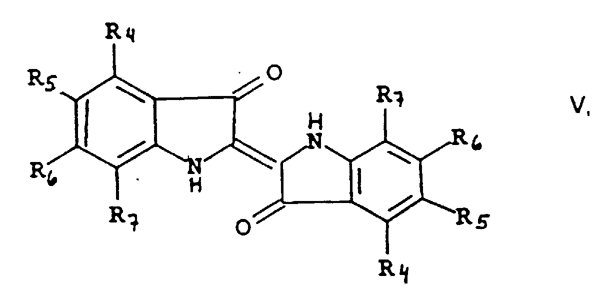 in which R₄, R₅, R₆ and R₇ have the meaning given in formula IV according to Claim 8, characterized in that an indoxyl derivative of the formula IV according to Claim 8 is prepared according to Claim 8 and oxidized in the customary manner to give the indigo derivative of the formula V.

## Revendications

1. Procédé de préparation d'un ester de glycine N-substitué ou d'un acide de glycine N-substitué répondant à la formule
R₁R₂N-CH₂-COOR I,
dans laquelle R signifie un groupe alkyle linéaire ou ramifié ayant de 1 à 10 atomes de carbone, et R₁ et R₂ signifient, indépendamment l'un de l'autre, l'hydrogène, un groupe phényle ou un groupe naphtyle non substitué ou substitué par un halogène, par des groupes alkyles ayant de 1 à 6 atomes de carbone ou par des groupes alcoxy ayant de 1 à 4 atomes de carbone, ou des groupes alkyles linéaires, ramifiés ou cycliques ayant de 1 à 22 atomes de carbone, qui peuvent être non substitués ou substitués par des groupes alcoxy ayant de 1 à 4 atomes de carbone ou des groupes phényle, R₁ et R₂ ne signifiant pas l'hydrogène en même temps,
caractérisé en ce qu'on fait réagir un hémi-acétal d'ester glyoxylique de formule
R₃O(OH)CH-COOR II,
dans laquelle R a la signification indiquée ci-dessus, et R₃ signifie un groupe alkyle ayant de 1 à 4 atomes de carbone, avec une amine de formule
R₁R₂NH III,
dans laquelle R₁ et R₂ ont les significations précédentes, dans un diluant, à des températures comprises entre 0°C et la température de reflux du diluant utilisé, et on traite le produit intermédiaire formé avec de l'hydrogène, sous pression, en présence d'un catalyseur d'hydrogénation et d'un diluant, et l'ester de glycine N-substitué de formule I se forme de cette manière, qu'on peut isoler du mélange de réaction si on le souhaite et qu'on transforme éventuellement en un sel ou en l'acide libre.

2. Procédé selon la revendication 1, caractérisé en ce qu'on utilise un composé de formule II dans laquelle R et R₃ sont identiques et signifient des groupes alkyles ayant de 1 à 4 atomes de carbone.

3. Procédé selon l'une des revendications 1 et 2, caractérisé en ce qu'on utilise un composé de formule III dans laquelle R₁ signifie un groupe phényle ou naphtyle non substitué ou substitué par un halogène, par des groupes alkyles ayant de 1 à 6 atomes de carbone ou par des groupes alcoxy ayant de 1 à 4 atomes de carbone, et R2 signifie l'hydrogène.

4. Procédé selon l'une des revendications 1 à 3, caractérisé en ce qu'on maintient une pression de 40 à 80 bars et une température de 20 à 130°C pendant l'hydrogénolyse.

5. Procédé selon l'une des revendications 1 à 4, caractérisé en ce qu'on utilise un composé de formule III dans laquelle R₁ signifie un groupe phényle ou naphtyle non substitué ou substitué par des groupes alkyles ayant de 1 à 4 atomes de carbone, et R₂ signifie l'hydrogène.

6. Procédé selon l'une des revendications 1 à 5, caractérisé en ce qu'on utilise de 1 à 1,5 mole de composé de formule III par mole de composé II.

7. Procédé selon l'une des revendications 1 à 6, caractérisé en ce qu'on utilise, comme diluant, un alcool aliphatique ayant de 1 à 4 atomes de carbone, dont la partie alkyle correspond aux parties alkyles de l'hémi-acétal d'ester glyoxylique utilisé.

8. Procédé de préparation d'un dérivé d'indoxyle de formule 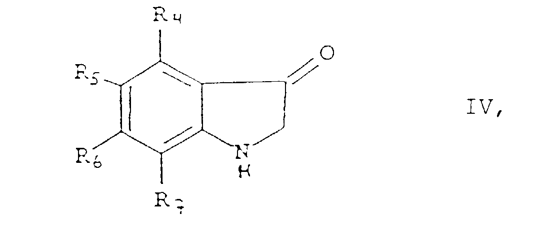 dans laquelle R₄, R₅, R₆ et R₇ signifient, indépendamment les uns des autres, l'hydrogène, un halogène, des groupes alkyles ayant de 1 à 6 atomes de carbone ou des groupes alcoxy ayant de 1 à 4 atomes de carbone, ou R₅ et R₆ ou R₆ et R₇ pris conjointement avec les deux atomes de carbone correspondants sur lesquels ils sont substitués signifient un noyau benzénique non substitué ou substitué par un halogène, par des groupes alkyles ayant de 1 à 6 atomes de carbone ou par des groupes alcoxy ayant de 1 à 4 atomes de carbone, qui est caractérisé en ce qu'on prépare, selon l'une des revendications 1 à 6, un ester de N-arylglycine de formule 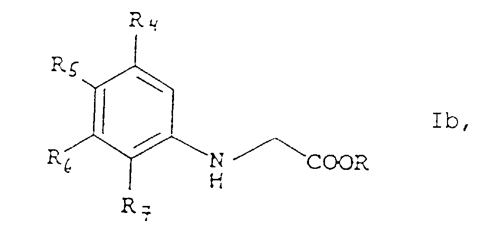 dans laquelle R a la signification indiquée pour la formule I selon la revendication 1, et R₄, R₅, R₆ et R₇ ont les significations indiquées ci-dessus, et on effectue sa cyclisation en indoxyle de formule IV, en présence d'un hydroxyde alcalin ou alcalino-terreux fondu, avec ou sans addition d'un amide alcalin, à des températures de 150 à 300°C.

9. Procédé selon la revendication 8, caractérisé en ce qu'on utilise un ester de N-arylglycine de formule la dans laquelle R signifie un groupe alkyle ayant de 1 à 4 atomes de carbone, et R₄, R₅, R₆ et R₇ signifient, indépendamment les uns des autres, l'hydrogène, un halogène, des groupes alkyles ayant de 1 à 4 atomes de carbone ou des groupes alcoxy ayant de 1 à 4 atomes de carbone.

10. Procédé de préparation d'un dérivé d'indigo répondant à la formule 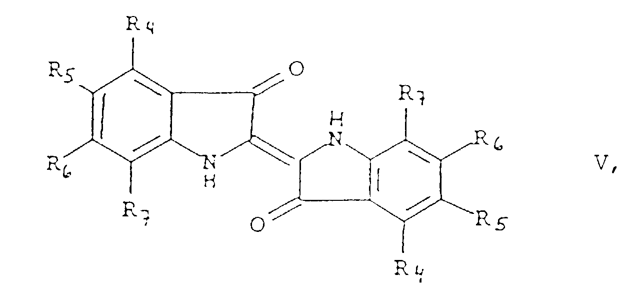 dans laquelle R₄, R₅, R₆ et R₇ ont les significations indiquées pour la formule IV selon la revendication 8, caractérisé en ce qu'on prépare un dérivé d'indoxyle de formule IV selon la revendication 8, conformément à la revendication 8, et on l'oxyde de façon usuelle en un dérivé d'indigo de formule V.
